# EUROPEAN PATENT SPECIFICATION

(11) **EP 3 878 347 B1**
(45) Date of publication and mention of the grant of the patent: **05.06.2024**
(21) Application number: 21170696.5
(22) Date of filing: 01.04.2017
(51) Int. Cl.: A61B 1/00, A61B 1/12, B65D 41/04, A61B 1/015

(54) **WATER BOTTLE CAP ASSEMBLIES FOR AN ENDOSCOPIC DEVICE**
WASSERFLASCHENKAPPENANORDNUNGEN FÜR EINE ENDOSKOPISCHE VORRICHTUNG
ENSEMBLES BOUCHON DE BOUTEILLE D'EAU POUR UN DISPOSITIF ENDOSCOPIQUE

(30) Priority: 01.04.2016 US 201662317162 P
(43) Date of publication of application: 15.09.2021
(62) Divisional of application: 17776890.0
(73) Proprietor: United States Endoscopy Group, Inc., Mentor, OH 44060 (US)
(72) Inventor: Mann, Gary E., Painesville, OH Ohio 44077 (US); Kaye, Christopher J., Berea, OH Ohio 44017 (US); Mrva, Joseph, Kirtland, OH Ohio 44094 (US); Hieber, Megan, Auburn Township, OH Ohio 44023 (US)
(74) Representative: Petraz, Gilberto Luigi

(56) References cited:
- WO-A1-01/85563
- JP-A- 2002 145 312
- US-A- 5 871 111
- US-A1- 2003 021 919
- US-A1- 2014 316 205
- US-B1- 6 485 412

## Description

### Background

The present disclosure relates to endoscope systems. More particularly, the present disclosure relates to water bottle cap assemblies, wherein the cap assemblies are operative for coupling a water bottle to an endoscope device in order to deliver sterilized water to the endoscope device.

Many invasive medical procedures that previously required major surgery are now performed using endoscopic instruments. For example, US 2014/0316205 A1 discloses an endoscopic instrument, and more particularly a combined tube set for a disposable water bottle for a medical instrument includes a cap with threads suitable for attachment to various water bottles. The combined tube set provides a first tube set for rinsing that includes an air and water tubes, and air/water connector The combined tube set also provides a second tube set for irrigation that includes an irrigation connector, backflow valve(s), and flexible tubing section. In some embodiments, the tube set can provide warm and/or humid gas to the endoscope. US 6,485,412 B1 also discloses an endoscopic instrument, and more particularly an adapter for a disposable water bottle for an endoscope. The adapter includes a cap with threads suitable for attachment to threads on a neck of the water bottle, an outer tube affixed to an opening in the cap, an inner tube extending through the outer tube so as to form an air passing annulus on the interior of the outer tube, and a fitting affixed to an end of the inner and outer tubes opposite the cap. The fitting is adapted for attachment to the air and water connections of the endoscope. A first gasket member is affixed to an interior surface of the cap and a second gasket member is affixed to the first gasket member such that the first gasket member is sandwiched between the second gasket member and the interior surface. An annular member is secured within a hole formed in the fitting and has a slot receiving a protrusion formed in the hole of the fitting. Additionally, US 5,871,111 A discloses an endoscopic instrument, and more particularly a screwable closure-cap with security against over-tightening. This cap possesses a ramp element on the end of the thread oriented towards the cap-base, said ramp element being able to engage with the thread-start of the container mouth in the case of over-tightening of the closure-cap. As a result, the cap-wall in the area of the ramp element will be forced outwards in the case of over-tightening so that jumping of the thread in this region will be facilitated. In this way, jumping of the thread on one side only--on the ramp element side--will be achieved when over-tightening is continued, so that ejection of the closure-cap will not be possible. Retention elements are arranged in the region of the seal. As a result of over-tightening and subsequent deformation of the region of the seal, said retention elements will make contact with the container mouth and the sealing-line will be interrupted. As a result, the container will be vented prior to jumping of the thread. Endoscopic instruments can provide an internal view of particular body parts, organs, or passages without requiring invasive surgery. Generally, an endoscopic instrument may include one or more channels through which miniaturized, flexible instruments can be inserted and advanced. The endoscope typically includes an elongated flexible insertion tube equipped at one end with an eyepiece or other viewing means and at the other end with an optical lens. The insertion tube transmits images or image-producing signals from the illuminated operative site to the viewing means to provide the instrument operator with full vision of the actions being performed at the instrument's working end.

The insertion tube of an endoscope also provides a flow passage for the delivery of fluid (e.g., liquid or gas) for irrigation, insufflation or other purposes. In conventional practice, it is necessary to provide a flow of sterile water across the optic lens to prevent the buildup of materials (e.g., surgical debris and body fluids) on the optic lens. This flow of water operates, in a sense, like a windshield wiper/washer assembly.

In common designs, an endoscopic instrument typically has a control body which is connected by a light guide tube to a light guide connector, which includes a plurality of connectors that can suitably receive various fittings. For example, the light guide connector can include a connector orifice that receives a grounding lug, a suction port, an air inlet, and a water inlet. As such, the air and water are delivered through the light guide connector, through the light guide tube and into the control body. Alternatively, the control body can also include a water port so as to allow water to be directly provided to the control body. Suitable valves are provided on the control body so as to control the flow of water through the control body and over the optic lens of the instrument.

### Summary

A water bottle cap assembly according to the present invention is as defined in annexed independent claim 1.

Other advantageous features are defined in annexed dependent claims.

Further features and advantages of the invention will become apparent from the following detailed description made with reference to the accompanying drawings.

### Brief Description of the Drawings

Features and advantages of the exemplary embodiments of the invention will become apparent from the following detailed description made with reference to the accompanying drawings.
Figure 1 is a perspective view of a water bottle cap assembly of one embodiment of the present subject matter;
Figure 2A is a top/left view of a cap with two ports of one embodiment of the present subject matter;
Figure 2B is a bottom/left view of the cap in Figure 2A;
Figure 2C is a cross-sectional view of the cap in Figure 2A;
Figure 3A is a cross-sectional view of a cap with single port of another embodiment of the present subject matter;
Figure 3B is a bottom/left view of the cap in Figure 3A;
Figure 3C is a cross-sectional view of the cap in Figure 3A;
Figure 4A is a top/left view of a cap with three ports of a third embodiment of the present subject matter;
Figure 4B is a bottom/left view of the cap in Figure 4A;
Figure 4C is a cross-sectional view of the cap in Figure 4A;
Figure 5 is a cross-sectional view of a multi-channel tube of one embodiment of the present subject matter;
Figures 6 is a perspective view of a passage connector of one embodiment of the present subject matter;
Figure 7A is a perspective view of a first adapter of one embodiment of the present subject matter;
Figure 7B is a cross-sectional view of the first adapter in Figure 7A;
Figure 8A is a perspective view of a first adapter of another embodiment of the present subject matter;
Figure 8B is a cross-sectional view of the first adapter in Figure 8A;
Figure 9A is a perspective view of a second adapter of one embodiment of the present subject matter;
Figure 9B is a perspective view of the second adapter in Figure 9A coupling to an air tube; and
Figure 9C is a cross-sectional view of the second adapter in Figure 9A.

### Detailed Description

This Detailed Description merely describes exemplary embodiments in accordance with the exemplary embodiments of the invention and is not intended to limit the scope of the invention or the claims in any way. Indeed, the invention is defined in the appended claims.

The exemplary embodiments of the invention will now be described with occasional reference to the exemplary embodiments of the invention. This general inventive concept may, however, be embodied in different forms and should not be construed as limited to the embodiments set forth herein. Rather, these embodiments are provided so that this disclosure will be thorough and complete, and will fully convey the scope of the exemplary embodiments of the invention to those skilled in the art.

Unless otherwise defined, all technical and scientific terms used herein have the same meaning as commonly understood by one of ordinary skill in the art encompassing the exemplary embodiments of the invention. The terminology set forth in this detailed description is for describing particular embodiments only and is not intended to be limiting of the exemplary embodiments of the invention. As used in this detailed description and the appended claims, the singular forms "a," "an," and "the" are intended to include the plural forms as well, unless the context clearly indicates otherwise.

Unless otherwise indicated, all numbers, such as for example, numbers expressing measurements or physical characteristics, used in the specification and claims are to be understood as being modified in all instances by the term "about." Accordingly, unless otherwise indicated, the numerical properties set forth in the specification and claims are approximations that may vary depending on the suitable properties sought to be obtained in embodiments of the invention. Notwithstanding that the numerical ranges and parameters setting forth the broad scope of the exemplary embodiments of the invention are approximations, the numerical values set forth in the specific examples are reported as precisely as possible. Any numerical values, however, inherently contain certain errors necessarily resulting from error found in their respective measurements.

There is a need for a water bottle cap assembly that is easily manufactured and cost effective. There is also a need for a water bottle cap assembly that is configured for use with a variety of endoscopic instruments, procedures (e.g., lens cleaning, secondary gas, and/or irrigation), and water sources. Moreover, there is a need for a water bottle cap assembly that is disposable so as to minimize cross contamination. Last but not least, there is a need for a multi-channel passage for conveying fluids between a water source and an endoscopic device.

Aspects of the present disclosure relate to water bottle cap assemblies configured for coupling a water bottle to an endoscopic device, e.g., a Pentax^{®} endoscope. As discussed below, the water bottle cap assembly comprises a cap configured to engage to a water bottle and a tubing assembly having at least one adaptor configured to engage the endoscopic device.

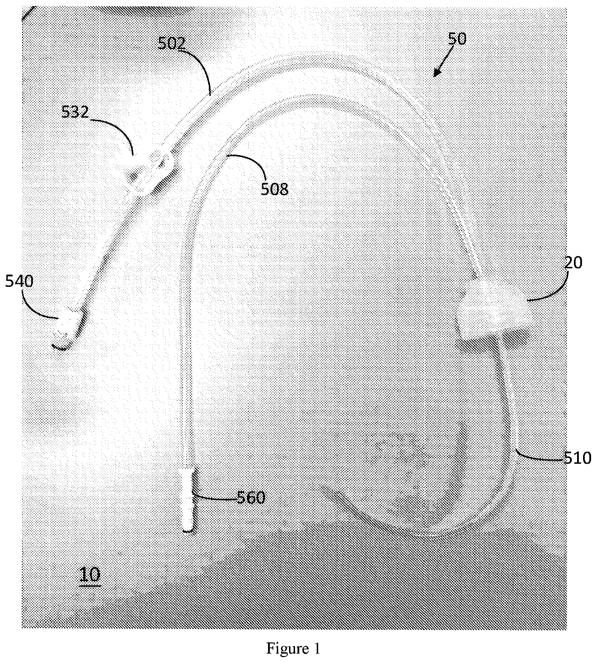

Referring now to the drawings, a water bottle cap assembly 10 is illustrated in Figure 1. The exemplary cap assembly 10 comprises a cap 20 and a passage assembly 50.

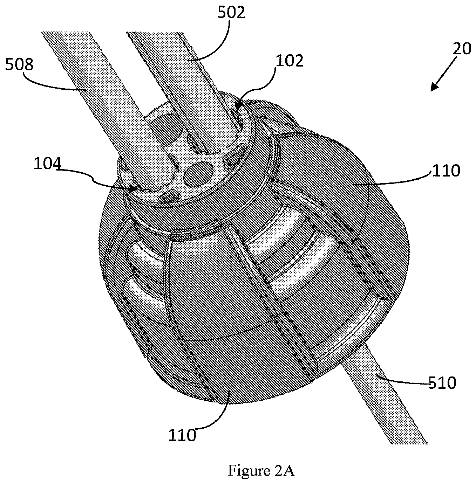

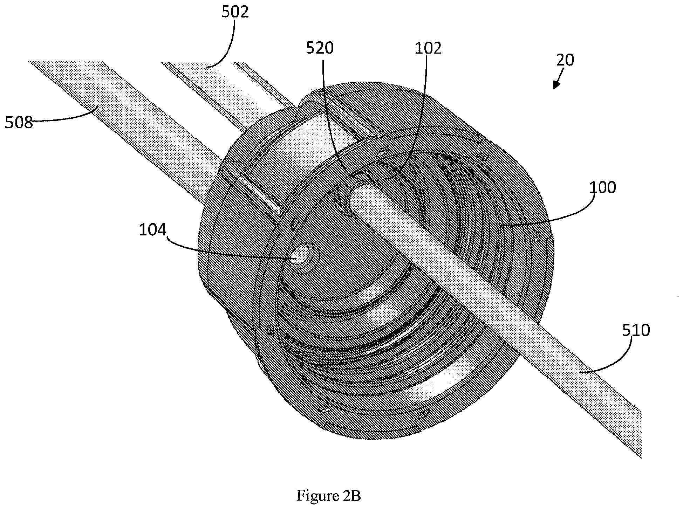

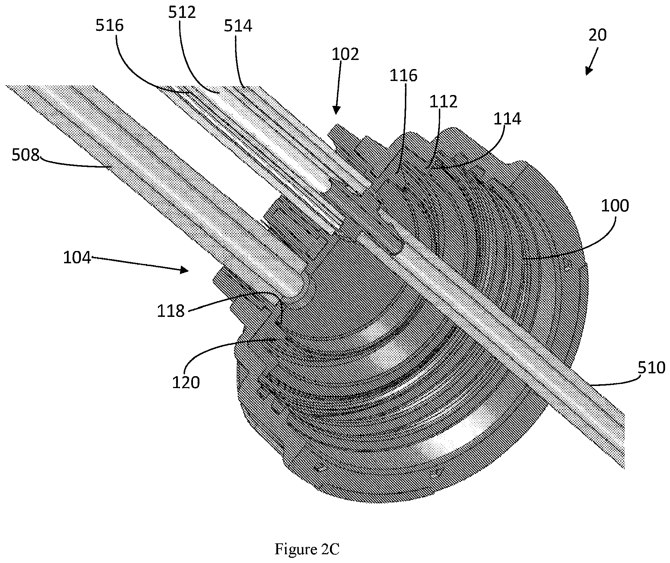

Figures 2A-2C show a cap 20 according to one embodiment of the present application. The cap 20 comprises a first port 102 and a second port 104 both extending outwardly from the exterior surface of the cap 20. The first and second ports 102, 104 extend a sufficient length so as to be configured to engage a passage member for providing or receiving a fluid to an endoscopic device. Each of the ports is configured to engage a respective passage member, wherein each passage member has a single or multiple channels. The passage member is configured to convey fluids between the water bottle and the endoscopic device (e.g., water and air or CO₂). As used herein, the term "fluid" is intended to encompass any material that may be described in relation to flow, such as a gas or a liquid, including solutions or other physical forms of a liquid or a gas that may include some concentration of a solid material in a dissolved, suspended, or otherwise mixed state that does not prevent flow of the liquid or gas.

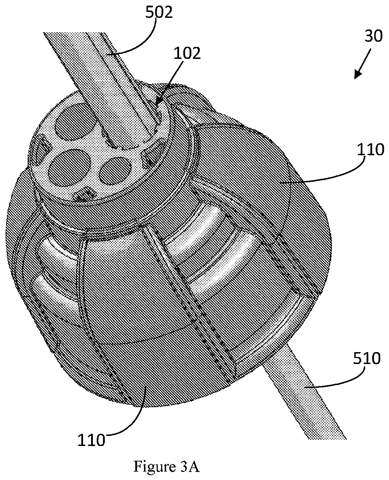

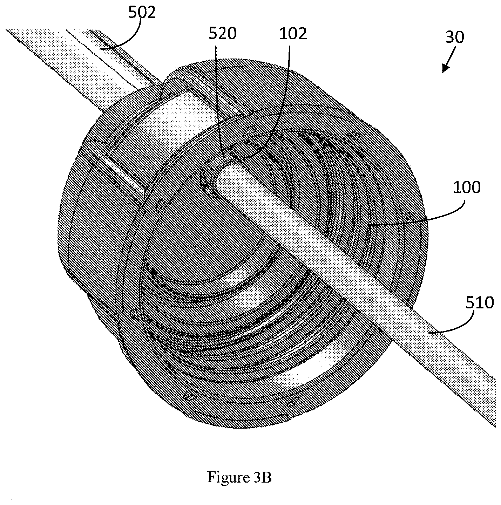

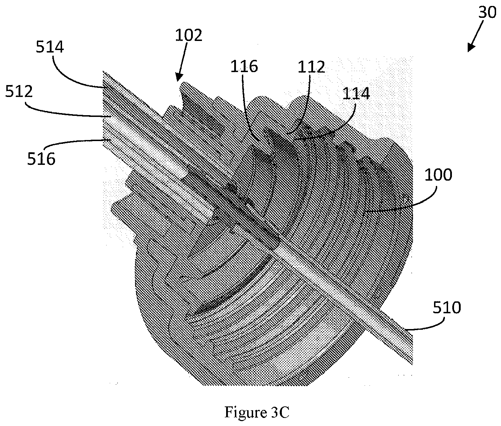

Figures 3A-3C show a cap 30 according to a second embodiment of the present application. The cap 30 comprises a first port 102 extending outwardly from the exterior surface of the cap 30. The first port 102 extends a sufficient length so as to be configured to engage a passage member for providing and/or receiving a fluid to an endoscopic device. The passage member has a single or multiple channels. The passage member is configured to convey fluids between the water bottle and the endoscopic device (e.g., water and air or CO₂).

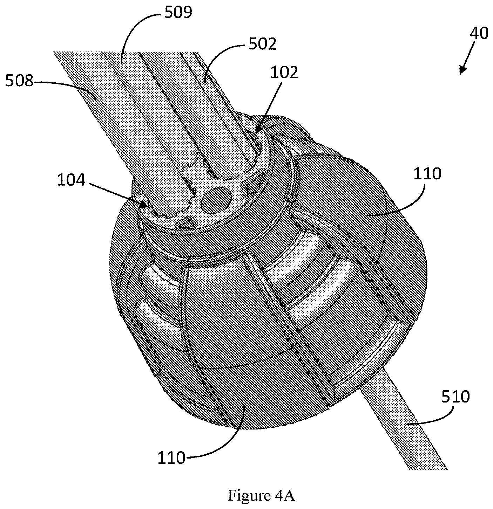

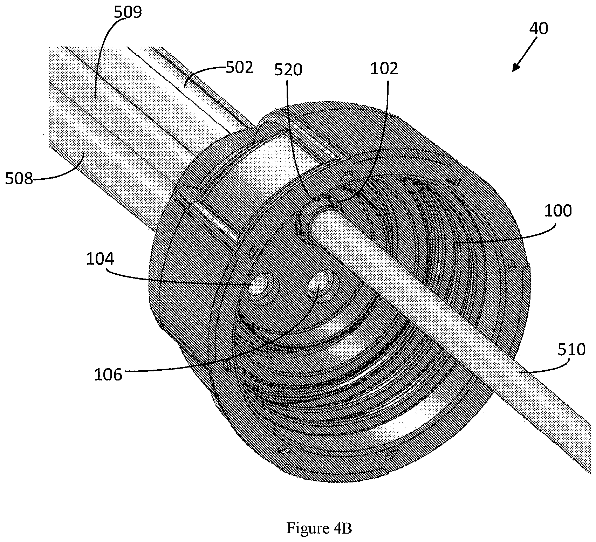

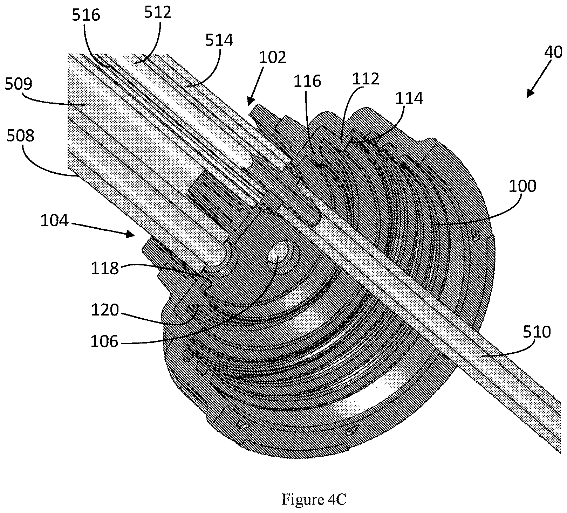

Figures 4A-4C show a cap 40 according to a third embodiment of the present application. The cap 40 comprises a first port 102, a second port 104, and a third port 106 all extending outwardly from the exterior surface of the cap 40. The first, second, and third ports extend a sufficient length so as to be configured to engagement a passage member for providing or receiving a fluid to an endoscopic device. In some embodiments, each of the ports is configured to engage a respective passage member, wherein each passage member has a single or multiple channels. The passage member is configured to convey fluids between the water bottle and the endoscopic device (e.g., water and air or CO₂). In some other embodiments, at least one of the ports is configured to be sealable and may be used to as an access port to inject desired substance into the water bottle.

Referring to Figures 2A-4C, the caps 20, 30, 40 comprise interior threads 100 for engaging external threads on a water bottle. A person skilled in the art may readily understand that the threads could be reversed if desired, i.e., external threads on the cap and internal threads in the water bottle. In this sense, the word "engage", when used in relation to a threaded engagement, is intended to mean a releasable arrangement wherein the various components can be engaged or coupled together by a screwing motion utilizing the threads and also may be detached by unscrewing. In some embodiments, an outer surface of the caps 20, 30, 40 may include a grip 110, such as raised ribs or a knurled surface, for facilitating rotation of the caps 20, 30, 40 by a user. In some embodiments, the caps 20, 30, 40 comprise four grips 110 on the outside surface of the caps 20, 30, 40.

In some embodiment, the caps 20, 30, 40 further comprise an annular sealing platform 112. The annular sealing platform 112 extends outwardly from the interior side wall of the caps 20, 30, 40. The annular sealing platform 112 has a certain cross sectional profile. In some embodiments, the annular sealing platform 112 comprises a substantially horizontal contact area 114. The horizontal contact area 114 provides a water-tight seal to a neck of a water bottle when the caps 20, 30, 40 are engaging the water bottle. In some embodiments, the ports 102, 104, 106 are disposed within the annular sealing platform 112.

In some embodiments, the caps 20, 30 40 further comprise a sealing ring 116. The sealing ring 116 extends outwardly from the interior top surface of the caps 20, 30, 40. The sealing ring 116 has a certain cross sectional profile. In some embodiments, the sealing ring 116 is a substantially tapered shape, in which the inner side wall 118 of the sealing ring 116 is substantially vertical and the outer side wall 120 of the sealing ring 116 is substantially inclined. In one embedment, the sealing ring 116 provides a water-tight seal to a neck of a water bottle when the caps 20, 30, 40 are engaging the water bottle. In another embodiment, the outer side wall 120 of the sealing ring 116 and the interior side wall of the cap 20, 30, 40 together provide a water-tight seal to a neck of a water bottle when the caps 20, 30, 40 are engaging the water bottle. In some embodiments, the ports 102, 104, 106 are disposed within the sealing ring 116.

In some embodiments, the sealing ring 116 is closer to the ports 102, 104, 106 than the annular sealing platform 112.

The caps 20, 30, 40 can be made of a plastic material, elastomeric material, and/or any suitable material or combination of materials. In one embodiment, the caps 20, 30, 40 comprise a first material and a second material. The second material is softer than the first material. The rigid first material prevents deformation of the cap. The resilient second material facilitates connection of the cap to the water bottle and to maintain a water-tight connection between the cap and the water bottle. Moreover, the resilient second material may be configured to absorb any slack while still maintaining a hermetic seal.

In some embodiments, the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106 are made of the second material. In some embodiments, the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106 are made in one-piece. In some embodiments, a combination of some of the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106 are made in one-piece. In some embodiments, the other parts of the caps 20, 30, 40 are made of the first material. In some embodiments, the second material is overmolded onto the first material and forms the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106. In some embodiments, the threads 100 are made of the first material. In some embodiments, the threads 100 are made of the second material. In some other embodiments, the second material and the first material are detachable.

Although the above embodiments only discloses single ports, two ports, and three ports on the caps, a person skilled in the art should understand that a cap may comprise more than three ports. In one embodiment, a first material made base of the cap has four ports. The second material is overmolded over the base and seals the unwanted ports during the manufacturing process. For example, if a cap with two ports is needed, the second material may be overmolded to the base with four ports and seal two of the four ports.

Back to Figures 2A-2C, the passage assembly 50 comprises a multi-channel passage member 502 and a single channel passage member, i.e., an air passage 508.

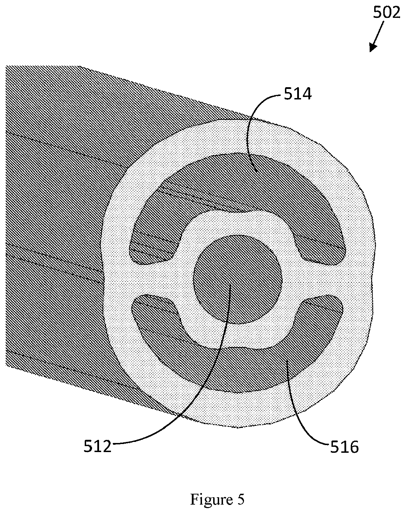

Further referring to the multi-channel passage member 502 in Figure 5, according to the invention, the multi-channel passage member 502 comprises a inner channel 512 and two C-shape outer channels 514, 516. In some embodiments, the multi-channel passage member 502 comprises a inner channel 512 and three C-shape outer channels. In some embodiments, the multi-channel passage member 502 comprises a inner channel 512 and four C-shape outer channels. In some embodiments, the inner channel 512 and the C-shape outer channels are substantially coaxial. In some embodiments, the outer channels are substantially same shaped or have substantially same cross-sectional area so that the multi-channel passage member 502 has a symmetrical geometry of its cross section. The multi-channel passage member 502 may be made of a variety of materials, including those that are water and CO₂ resistant. In some embodiments, the multi-channel passage member 502 is made in one-piece. A person skilled in the art may understand that the multi-channel passage member 502 may has more than four channels as long as the multi-channel passage member 502 has a symmetrical geometry of its cross section.

Back to Figures 2A-2C, the first port 102 is configured to couple to the multi-channel passage member 502. The second port 104 is configured to receive the air passage 508. The multi-channel passage member 502 is coupled to the first port 102 in a fluid-tight manner, such as using a force-fit connection. In another embodiment, a portion of the outside of the multi-channel passage member 502 is glued to the inside surface of the first port 102. A person skilled in the art may understand that other suitable connection method may be used here. Similarly, the air passage 508 is secured to the second port 104 so as to be in fluid-tight communication.

In some embodiments, the inner channel 512 extends beyond the outer channels 514, 516 and into the water bottle. In some other embodiments, such as the embodiment shown in Figures 2A-C, the inner channel 512 has a similar length as the outer channels 514, 516 and does not extend into the water bottle. A water passage 510 is configured to be disposed inside the cap 20 and is configured to couple to the inner channel 512 via a channel connector 520. As such, the first port 102 is configured to provide air to the endoscopic device through the outer channels 514, 516. The first port 102 is also configured to provide water to the endoscopic device through the inner channel 512. The second port 104 is configured to receive air through the air passage 508 for charging the water within the bottle. Thus, air can be provided into the water bottle to pressurize the water to convey air and/or water to the endoscopic device.

Referring to Figures 3A-3C, the passage assembly 50 comprises a multi-channel passage member 502 shown in Figures 2A-2C. The first port 102 is configured to couple to the multi-channel passage member 502 as shown in Figure 2A-C. In some embodiments, the first port 102 is configured to receive air or CO₂ from a pressurized air or CO₂ source through the outer channels 514, 516 for charging the water within the bottle and to provide water to the endoscopic device through the inner channel 512. Thus, air or CO₂ can be provided into the water bottle to pressurize the water to convey water to the endoscopic device.

Referring to Figures 4A-4C, the passage assembly 50 comprises a multi-channel passage member 502 shown in Figures 2A-2C. The first port 102 is configured to couple to the multi-channel passage member 502 as shown in Figure 2A-C. The second port 104 is configured to couple to the air passage 508 as shown in Figure 2A-C. The third port 106 is configured to couple to a third passage 509. In some embodiments, the third passage 509 has the same configurations as the air passage 508. As such, the first port 102 is configured to provide air to the endoscopic device through the outer channels 514, 516. The first port 102 is also configured to provide water to the endoscopic device through the inner channel 512. The second port 104 is configured to receive air through the air passage 508 for charging the water within the bottle. The third port 106 is configured to provide water from the bottle for, but not limited to, irrigation or cleaning purposes. Thus, air can be provided into the water bottle to pressurize the water to convey air and/or water to the endoscopic device.

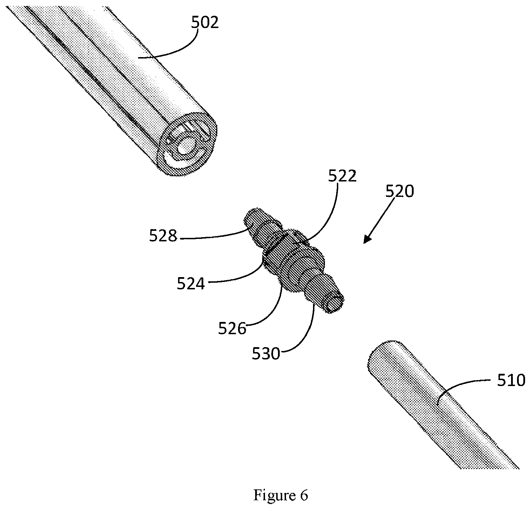

Referring to Figure 6, the channel connector 520 comprises a hollow body 522 with two open ends, two discs 524, 526 disposed at a middle portion of the outer surface of the hollow body 522, and two barbs 528, 530 near each end of the hollow body 522. In some embodiments, the channel connector 520 comprises only one disc 524. The discs 524, 526 ensure both the inner channel 512 and the water passage 510 be able to engage the channel connector 520 at a desired contact length. The discs do not substantially obstruct the outer channels 514, 516. When the water passage 510 is coupled to the inner channel 512 via the channel connector 520, the barbs 528, 530 are used to form the fluid-tight connections.

A pinch clamp 532 may also be provided on the multi-channel passage member 502 that is configured to close off fluid communication between the water bottle and the endoscopic device.

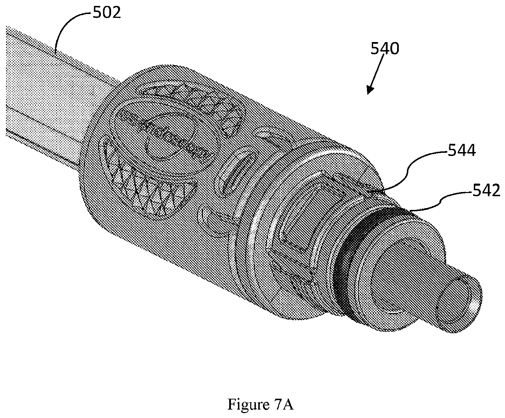

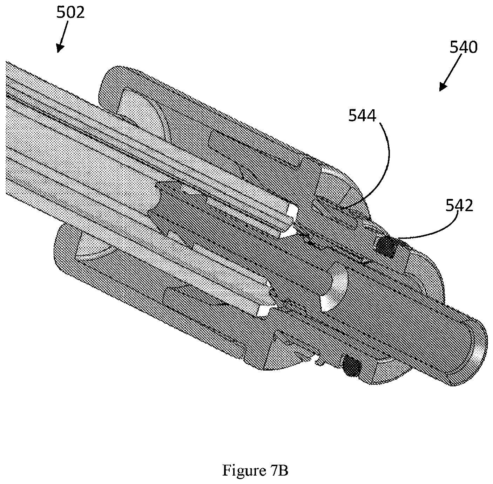

A first embodiment of the first adapter 540 is shown in Figures 7A-7B. The first adapter 540 is configured to couple to the distal end of the multi-channel passage member 502. An o-ring 542 is provided at the distal end of the first adapter 540 for sealing. A molded retention feature 544 is provided in the first adapter 540 and is configured to flex out of the way and snaps back in.

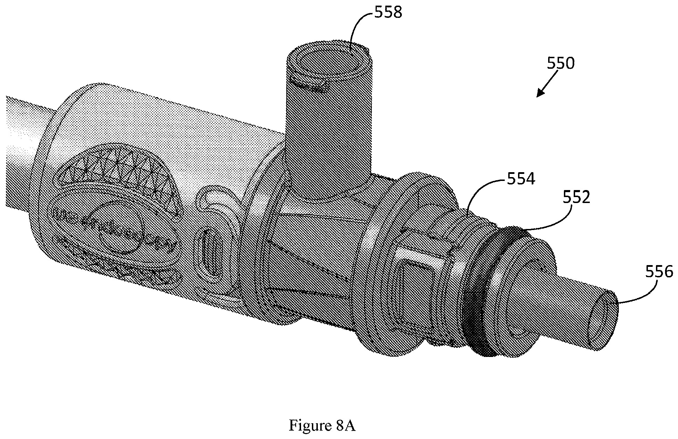

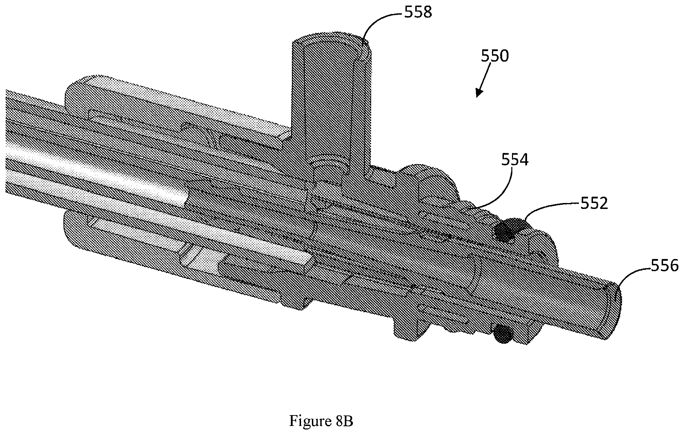

Referring to Figures 8A-8B, another embodiment of the first adapter 550 is shown. The first adapter 550 is configured to form a pathway by which CO₂ can be used for insufflation instead of air. The first adapter 550 has the similar o-ring 552 and molded retention feature 554. The first adapter 550 has a water output 556 and a CO₂ input 558. In one embodiment, the CO₂ input 558 comprises a removable seal cover (not shown). The removable seal cover is configured to seal the CO₂ input 558 when CO₂ is not in use.

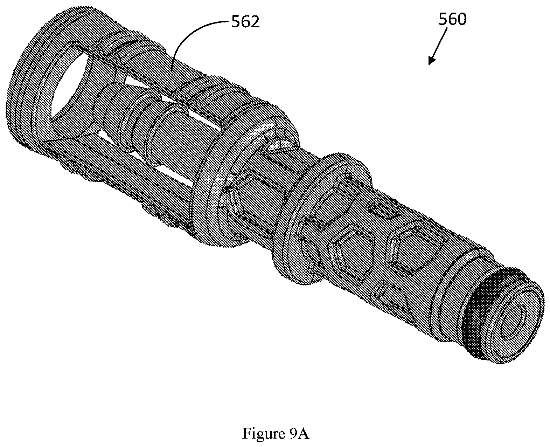

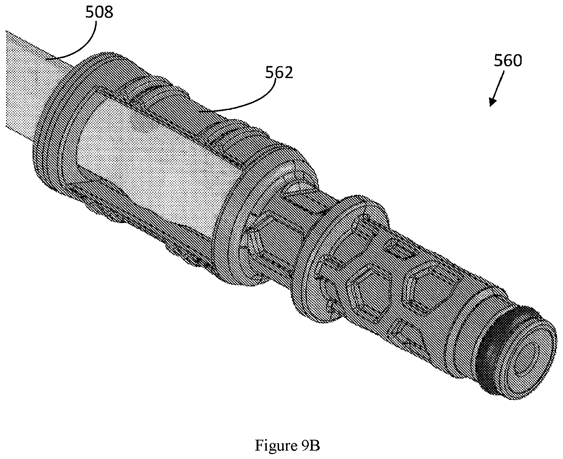

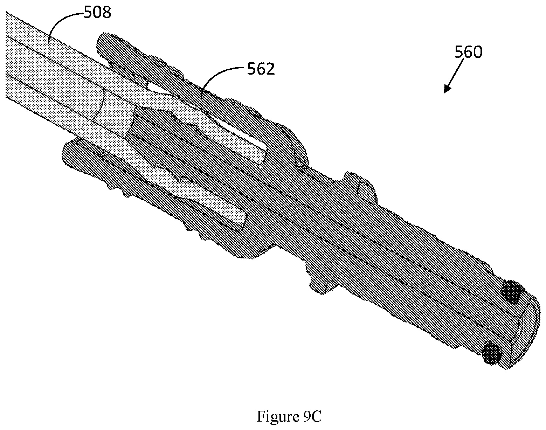

Referring to Figure 9A-9B, a second adapter 560 is shown. The second adapter 560 is configured to couple to the distal end of the air passage 508 and is configured to couple to the Pentax video processor to allow air to flow into the water bottle. The second adapter 560 comprises a cage grip 562. The cage grip 562 at least partially covers the connection portion between the second adapter and the air passage 508. The cage grip 562 helps a user to pull off the second adapter 560 from the video processor without directly pulling the air passage 508.

The present subject matter discloses that a water bottle cap (20) comprises: at least one port, extending outwardly from a exterior surface of the cap, wherein the port extends a predetermined length and is configured to engage a passage member for providing or receiving a fluid to an endoscopic device, interior threads, configured to engage external threads on a water bottle, and an annular sealing platform (112), extending outwardly from a interior side wall of the cap, wherein the annular sealing platform comprises a substantial horizontal contact area (114), configured to provide a water-tight seal to a neck of a water bottle when the cap is engaging the water bottle. The port is disposed within the annular sealing platform (112). The water bottle cap further comprises a sealing ring (116), extending outwardly from an interior top surface of the cap and providing a water-tight seal to a neck of a water bottle when the cap is engaging the water bottle. The sealing ring (116) is a substantially tapered shape. An inner side wall (118) of the sealing ring is substantially vertical, and an outer side wall (120) of the sealing ring is substantially inclined. The outer side wall of the sealing ring and an interior side wall of the cap are configured to provide a water-tight seal to a neck a water bottle when the cap is engaging the water bottle. The port is disposed with the sealing ring (116). The sealing ring (116) is closer to the port than the annular sealing platform (112). The water bottle cap further comprises a grip (110), disposed on an outer surface of the cap. The cap is made of a first material and a second material, wherein the second material is softer than the first material. At least the annular sealing platform (112), the interior top surface of the cap, an interior side wall of the cap, and inside and outside surfaces of the port are made of a second material softer than other parts of the cap made of a first material. At least the annular sealing platform (112), the sealing ring (116), the interior top surface of the cap, the interior side wall of the cap, and inside and outside surfaces of the port are made of a second material softer than other parts of the cap made of a first material. At least the grip (110), the annular sealing platform (112), the interior top surface of the cap, an interior side wall of the cap, and inside and outside surfaces of the port are made of a second material softer than other parts of the cap made of a first material. At least the grip (110), the annular sealing platform (112), the sealing ring (116), the interior top surface of the cap, the interior side wall of the cap, and inside and outside surfaces of the port are made of a second material softer than other parts of the cap made of a first material. The second material is made in one-piece. The second material is overmolded onto the first material.

The present subject matter discloses that a water bottle cap assembly comprises a water bottle cap (20), comprising at least one port, extending outwardly from a exterior surface of the cap, and a passage assembly (50), comprising a multi-channel passage member (502), wherein the port extends a predetermined length and is configured to engage the passage assembly in a fluid-tight manner for providing or receiving a fluid to an endoscopic device. The multi-channel passage member (502) comprises an inner channel (512) and at least one C-shape outer channel. The multi-channel passage member comprises at least two C-shape outer channels (514, 516). Each outer channel has a substantially same cross-sectional area. The multi-channel passage member (502) has a symmetrical geometry of its cross section. The inner channel and the outer channel are coaxial. The multi-channel passage member (502) is made in one-piece. The multi-channel passage member (502) is made of water and CO₂ resistant material. The inner channel (512) extends beyond the outer channels (514, 516) and passes through the cap. The water bottle cap assembly further comprising: a second passage (510), and a channel connector (520), wherein the channel connector (520) comprises: a hollow body (522) with two open ends, two barbs (528, 530), disposed near each open end of the hollow body and configured to form the fluid-tight connections when the second passage is coupled to the inner channel via the channel connector, wherein the inner channel (512) has a similar length as the outer channels (514, 516). The channel connector (520) further comprises at least one disc (524, 526), disposed at a middle portion of the outer surface of the hollow body, wherein the disc is configured to allow the inner channel (512) and the water passage (510) to engage the channel connector at a predetermined contact length.

The present subject matter discloses that an adapter (540, 550) for coupling a multi-channel passage member to an endoscopic device comprises: a first connection portion , configured to couple with the multi-channel passage, a second connection portion, configured to couple with the endoscopic device, wherein the first and second connection portions have at least one fluid communication, an o-ring (542, 552), disposed on the second connection portion and configured to provide sealing between the adapter and the endoscopic device, and a molded retention (544, 554), disposed on the second connection portion and configured to flex out of the way and snaps back in to the endoscopic device. The adapter (550) further comprising a CO₂ input port (558), disposed on the second connection portion, wherein the CO2 input port has a fluid communication with one channel of the multi-channel passage, when the adapter is coupled with the multi-channel passage.

The present subject matter discloses that an adapter (560) for coupling an air passage to a video processor, comprises: a first connection portion, configured to couple with the air passage, a second connection portion, configured to couple with the video processor, and a cage grip (562), partially covering the first connection portion, wherein the cage grip (562) is configured to prevent a user from directly pulling the air passage.

While various features of the exemplary embodiments of the invention are described and illustrated herein in the context of various exemplary embodiments, the invention itself and preferred embodiments thereof are defined in the claims.

## Claims

1. A water bottle cap assembly (10, 20, 50), comprising:
at least one port (102), extending outwardly from an exterior surface of the cap, a multi-channel passage member (502) for providing or receiving a fluid to an endoscopic device, wherein the multi-channel passage member includes an inner channel (512) and a first and second c-shaped outer channel (514, 516), wherein the port extends a predetermined length and is configured to engage the multi-channel passage member,
a plurality of interior threads, configured to engage a plurality of external threads on a water bottle,
an annular sealing platform (112), extending outwardly from an interior side wall of the cap, wherein the annular sealing platform comprises a substantial horizontal contact area (114), configured to provide a water-tight seal to a neck of a water bottle when the cap is engaging the water bottle, and
a channel connector (520) configured to couple the inner channel to a water passage (510) disposed inside the water bottle cap for distributing water from the water bottle to the endoscopic device,
wherein the first and second outer channels do not extend into the water bottle, and
wherein the at least one port is configured to provide water to an endoscopic device through the inner channel and a pressurized air to the endoscopic device through at least one of the outer channels.

2. The water bottle cap assembly of claim 1, wherein the port is disposed within the annular sealing platform (112).

3. The water bottle cap assembly of claim 1 further comprising a sealing ring (116), extending outwardly from an interior top surface of the cap and providing a water-tight seal to a neck of a water bottle when the cap is engaging the water bottle.

4. The water bottle cap assembly of claim 3, wherein the sealing ring (116) is a substantially tapered shape.

5. The water bottle cap assembly of claim 3, wherein an inner side wall (118) of the sealing ring is substantially vertical, and an outer side wall of the sealing ring is substantially inclined.

6. The water bottle cap assembly of claim 3, wherein an outer side wall of the sealing ring and an interior side wall of the cap are configured to provide a water-tight seal to a neck of a water bottle when the cap is engaging the water bottle.

7. The water bottle cap assembly of claim 3, wherein the port is disposed with the sealing ring (116).

8. The water bottle cap assembly of claim 3, wherein the sealing ring (116) is closer to the port than the annular sealing platform.

9. The water bottle cap assembly of claim 1 or 3, further comprising a grip (110), disposed on an outer surface of the cap.

10. The water bottle cap assembly of claim 1, wherein the cap is made of a first material and a second material, wherein the second material is softer than the first material.

11. The water bottle cap assembly of claim 1, wherein at least the annular sealing platform the interior top surface of the cap, an interior side wall of the cap, and inside and outside surfaces of the port are made of a second material softer than other parts of the cap made of a first material.

12. The water bottle cap assembly of claim 3, wherein at least the annular sealing platform the sealing ring, the interior top surface of the cap, the interior side wall of the cap, and inside and outside surfaces of the port are made of a second material softer than other parts of the cap made of a first material.

13. The water bottle cap assembly of claim 9, wherein at least the grip, the annular sealing platform, the interior top surface of the cap, an interior side wall of the cap, and inside and outside surfaces of the port are made of a second material softer than other parts of the cap made of a first material.

14. The water bottle cap assembly of any of the preceding claims 10 to 13, wherein the second material is overmolded onto the first material.

15. The water bottle cap assembly according to any one of the preceding claims,
wherein the channel connector includes a hollow body with two open ends and two barbs (528, 530) near each open end, and at least one disc (524) to ensure engagement between the channel connector, the inner channel, and the water passage, and wherein the at least one disc does not substantially obstruct the first and second outer channels.

## Patentansprüche

1. Wasserflaschenkappenanordnung (10, 20, 50), umfassend:
mindestens einen Anschluss (102), der sich von einer außenseitigen Oberfläche der Kappe nach außen erstreckt, ein mehrkanaliges Durchlassteil (502) zum Bereitstellen oder Aufnehmen eines Fluids zu einer endoskopischen Vorrichtung, wobei das mehrkanalige Durchlassteil einen inneren Kanal (512) und einen ersten und zweiten C-förmigen äußeren Kanal (514, 516) beinhaltet, wobei sich der Anschluss über eine vorbestimmte Länge erstreckt und konfiguriert ist, um mit dem mehrkanaligen Durchlassteil in Eingriff zu kommen,
eine Vielzahl von innenseitigen Gewindegängen, die konfiguriert ist, um mit einer Vielzahl von außenseitigen Gewindegängen an einer Wasserflasche in Eingriff zu kommen,
eine ringartige Dichtfläche (112), die sich von einer innenseitigen Seitenwand der Kappe nach außen erstreckt, wobei die ringartige Dichtfläche einen im Wesentlichen horizontalen Kontaktbereich (114) umfasst, der konfiguriert ist, um eine wasserdichte Abdichtung zu einem Hals einer Wasserflasche bereitzustellen, wenn die Kappe mit der Wasserflasche in Eingriff ist, und
einen Kanalverbinder (520), der konfiguriert ist, um den inneren Kanal an einen Wasserdurchlass (510) zu koppeln, der innerhalb der Wasserflaschenkappe angeordnet ist, um Wasser von der Wasserflasche zu der endoskopischen Vorrichtung zu verteilen,
wobei sich der erste und zweite äußere Kanal nicht in die Wasserflasche hinein erstrecken, und
wobei der mindestens eine Anschluss konfiguriert ist, um Wasser für eine endoskopische Vorrichtung durch den inneren Kanal und eine Druckluft für die endoskopische Vorrichtung durch mindestens einen der äußeren Kanäle bereitzustellen.

2. Wasserflaschenkappenanordnung nach Anspruch 1, wobei der Anschluss innerhalb der ringartigen Dichtfläche (112) angeordnet ist.

3. Wasserflaschenkappenanordnung nach Anspruch 1, ferner umfassend einen Dichtring (116), der sich von einer innerseitigen Deckfläche der Kappe nach außen erstreckt und eine wasserdichte Abdeckung zu einem Hals einer Wasserflasche bereitstellt, wenn die Kappe mit der Wasserflasche in Eingriff ist.

4. Wasserflaschenkappenanordnung nach Anspruch 3, wobei der Dichtring (116) eine im Wesentlichen verjüngte Form aufweist.

5. Wasserflaschenkappenanordnung nach Anspruch 3, wobei eine innere Seitenwand (118) des Dichtrings im Wesentlichen vertikal ist und eine äußere Seitenwand des Dichtrings im Wesentlichen geneigt ist.

6. Wasserflaschenkappenanordnung nach Anspruch 3, wobei eine äußere Seitenwand des Dichtrings und eine innere Seitenwand der Kappe konfiguriert sind, um eine wasserdichte Abdichtung zu einem Hals einer Wasserflasche bereitzustellen, wenn die Kappe mit der Wasserflasche in Eingriff ist.

7. Wasserflaschenkappenanordnung nach Anspruch 3, wobei der Anschluss mit dem Dichtring (116) angeordnet ist.

8. Wasserflaschenkappenanordnung nach Anspruch 3, wobei der Dichtring (116) näher an dem Anschluss als die ringartige Dichtfläche ist.

9. Wasserflaschenkappenanordnung nach Anspruch 1 oder 3, ferner umfassend eine Griffstruktur (110), die an einer äußeren Oberfläche der Kappe angeordnet ist.

10. Wasserflaschenkappenanordnung nach Anspruch 1, wobei die Kappe aus einem ersten Material und einem zweiten Material hergestellt ist, wobei das zweite Material weicher als das erste Material ist.

11. Wasserflaschenkappenanordnung nach Anspruch 1, wobei mindestens die ringartige Dichtfläche, die innenseitige Deckfläche der Kappe, eine innere Seitenwand der Kappe und eine innere und äußere Oberfläche des Anschlusses aus einem zweiten Material hergestellt sind, das weicher ist als andere Teile der Kappe, die aus einem ersten Material hergestellt sind.

12. Wasserflaschenkappenanordnung nach Anspruch 3, wobei mindestens die ringartige Dichtfläche, der Dichtring, die innenseitige Deckfläche der Kappe, die innere Seitenwand der Kappe und die innere und äußere Oberfläche des Anschlusses aus einem zweiten Material hergestellt sind, das weicher ist als andere Teile der Kappe, die aus einem ersten Material hergestellt sind.

13. Wasserflaschenkappenanordnung nach Anspruch 9, wobei mindestens die Griffstruktur, die ringartige Dichtfläche, die innenseitige Deckfläche der Kappe, eine innere Seitenwand der Kappe und eine innere und äußere Oberfläche des Anschlusses aus einem zweiten Material hergestellt sind, das weicher ist als andere Teile der Kappe, die aus einem ersten Material hergestellt sind.

14. Wasserflaschenkappenanordnung nach einem der vorhergehenden Ansprüche 10 bis 13, wobei das zweite Material auf das erste Material überspritzt ist.

15. Wasserflaschenkappenanordnung nach einem der vorhergehenden Ansprüche,
wobei der Kanalverbinder einen Hohlkörper mit zwei offenen Enden und zwei Widerhaken (528, 530) nahe jedem offenen Ende und mindestens eine Scheibe (524) beinhaltet, um einen Eingriff zwischen dem Kanalverbinder, dem inneren Kanal und dem Wasserdurchlass zu gewährleisten, und wobei die mindestens eine Scheibe den ersten und zweiten äußeren Kanal nicht wesentlich behindert.

## Revendications

1. Ensemble bouchon de bouteille d'eau (10, 20, 50), comprenant :
au moins un orifice (102), s'étendant vers l'extérieur depuis une surface extérieure du bouchon, un organe de passage multicanal (502) pour fournir un fluide à un dispositif endoscopique ou recevoir un fluide depuis celui-ci, dans lequel l'organe de passage multicanal comprend un canal interne (512) et un premier et un second canal externe en forme de c (514, 516), dans lequel l'orifice s'étend sur une longueur prédéterminée et est configuré de façon à venir en prise avec l'organe de passage multicanal,
une pluralité de filetages intérieurs, configurés pour venir en prise avec une pluralité de filetages extérieurs sur une bouteille d'eau,
une plate-forme d'étanchéité annulaire (112), s'étendant vers l'extérieur à partir d'une paroi latérale intérieure du bouchon, dans lequel la plate-forme d'étanchéité annulaire comprend une zone de contact horizontale substantielle (114), configurée pour fournir une étanchéité à l'eau à un goulot d'une bouteille d'eau lorsque le bouchon est en prise avec la bouteille d'eau, et
un raccord de canal (520) configuré pour raccorder le canal interne à un passage d'eau (510) disposé à l'intérieur du bouchon de bouteille d'eau pour distribuer l'eau de la bouteille d'eau au dispositif endoscopique,
dans lequel les premier et second canaux externes ne s'étendent pas dans la bouteille d'eau, et
dans lequel l'au moins un orifice est configuré pour fournir de l'eau à un dispositif endoscopique par le canal interne et de l'air sous pression au dispositif endoscopique par au moins l'un des canaux externes.

2. Ensemble bouchon de bouteille d'eau selon la revendication 1, dans lequel l'orifice est disposé à l'intérieur de la plate-forme d'étanchéité annulaire (112).

3. Ensemble bouchon de bouteille d'eau selon la revendication 1, comprenant en outre un anneau d'étanchéité (116) s'étendant vers l'extérieur depuis une surface supérieure intérieure du bouchon et fournissant une étanchéité au goulot d'une bouteille d'eau lorsque le bouchon est en prise avec la bouteille d'eau.

4. Ensemble bouchon de bouteille d'eau selon la revendication 3, dans lequel l'anneau d'étanchéité (116) est de forme sensiblement effilée.

5. Ensemble bouchon de bouteille d'eau selon la revendication 3, dans lequel une paroi latérale intérieure (118) de l'anneau d'étanchéité est sensiblement verticale, et une paroi latérale extérieure de l'anneau d'étanchéité est sensiblement inclinée.

6. Ensemble bouchon de bouteille d'eau selon la revendication 3, dans lequel une paroi latérale extérieure de l'anneau d'étanchéité et une paroi latérale intérieure du bouchon sont configurées pour fournir une étanchéité à l'eau à un goulot d'une bouteille d'eau lorsque le bouchon est en prise avec la bouteille d'eau.

7. Ensemble bouchon de bouteille d'eau selon la revendication 3, dans lequel l'orifice est disposé avec l'anneau d'étanchéité (116) .

8. Ensemble bouchon de bouteille d'eau selon la revendication 3, dans lequel l'anneau d'étanchéité (116) est plus proche de l'orifice que la plate-forme d'étanchéité annulaire.

9. Ensemble bouchon de bouteille d'eau selon la revendication 1 ou 3, comprenant en outre une poignée (110) disposée sur une surface extérieure du bouchon.

10. Ensemble bouchon de bouteille d'eau selon la revendication 1, dans lequel le bouchon est constitué d'un premier matériau et d'un second matériau, dans lequel le second matériau est plus mou que le premier matériau.

11. Ensemble bouchon de bouteille d'eau selon la revendication 1, dans lequel au moins la plate-forme d'étanchéité annulaire, la surface supérieure intérieure du bouchon, une paroi latérale intérieure du bouchon et les surfaces intérieure et extérieure de l'orifice sont constituées d'un second matériau plus mou que d'autres parties du bouchon constituées d'un premier matériau.

12. Ensemble bouchon de bouteille d'eau selon la revendication 3, dans lequel au moins la plate-forme d'étanchéité annulaire, l'anneau d'étanchéité, la surface supérieure intérieure du bouchon, une paroi latérale intérieure du bouchon et les surfaces intérieure et extérieure de l'orifice sont constituées d'un second matériau plus mou que d'autres parties du bouchon constituées d'un premier matériau.

13. Ensemble bouchon de bouteille d'eau selon la revendication 9, dans lequel au moins la poignée, la plate-forme d'étanchéité annulaire, la surface supérieure intérieure du bouchon, une paroi latérale intérieure du bouchon et les surfaces intérieure et extérieure de l'orifice sont constituées d'un second matériau plus mou que d'autres parties du bouchon constituées d'un premier matériau.

14. Ensemble bouchon de bouteille d'eau selon l'une quelconque des revendications précédentes 10 à 13, dans lequel le second matériau est surmoulé sur le premier matériau.

15. Ensemble bouchon de bouteille d'eau selon l'une quelconque des revendications précédentes,
dans lequel le raccord de canal comprend un corps creux avec deux extrémités ouvertes et deux pointes (528, 530) à proximité de chaque extrémité ouverte, et au moins un disque (524) pour assurer la mise en prise entre le raccord de canal, le canal interne et le passage d'eau, et dans lequel l'au moins un disque n'obstrue sensiblement pas les premier et second canaux externes.
